# EUROPEAN PATENT SPECIFICATION

(11) **EP 1 792 623 B1**
(45) Date of publication and mention of the grant of the patent: **10.03.2010**
(21) Application number: 05014902.0
(22) Date of filing: 08.07.2005
(51) Int. Cl.: A61K 36/28, A61P 25/16, A61P 25/28

(54) **Use of extracts and constituents of leontopodium as enhancers of cholinergic function**
Verwendung von Extrakten und Inhaltsstoffen aus Leontopodium zur Steigerung der cholinergen Funktion
Utilisation des extraits et des constituants du leontopodium pour l'amélioration de la fonction cholinergique

(43) Date of publication of application: 06.06.2007
(73) Proprietor: Leopold-Franzens-Universität Innsbruck, 6020 Innsbruck (AT)
(72) Inventor: PRAST, Helmut, 6020 Innsbruck (AT)
(74) Representative: Grünecker, Kinkeldey, Stockmair & Schwanhäusser Anwaltssozietät

(56) References cited:
- WO-A-02/34715
- SCHWAIGER STEFAN ET AL: "New constituents of Leontopodium alpinum and their in vitro leukotriene biosynthesis inhibitory activity" PLANTA MEDICA, vol. 70, no. 10, October 2004 (2004-10), pages 978-985, XP009051973 ISSN: 0032-0943

## Description

The present invention relates to the use of a plant extract from at least one *Leontopodium* species for the preparation of a medicament for the treatment of diseases which can be modulated by the selective inhibition of the enzyme acetylcholinesterase and/or increase of acetylcholine concentration at the cholinergic synapse when the disease is selected from Alzheimer's disease, vascular dementia, dementia with Lewis bodies, dementia associated with Parkinson's disease and the Wemicke-Korsakoff-Syndrome..

*Leontopodium alpinum* Cass. (Asteraceae), also known as "Edelweiss" grows on rocky and grassy slopes, mostly on limestone at altitudes from 220 to 3140 m. It is found in the mountainous regions of Europe, especially in the Alps, Carpatian, the mountains of the Balkan peninsular, the Tatra and the Pyrenees.

In European folk medicine, extracts of this plant are used for the treatment of abdominal aches, diarrhoea, angina, bronchitis, cancer, dysentery and fever for humans as well as for live-stock (G. Hegi, Illustrierte Flora von Mitteleuropa, Volume 6 (1935), Vienna, p. 158-461; T. Bitschnau, diploma theses 1991, University of Vienna; K. Kiene, diploma thesis 1992, University of Vienna; E. Knechtl, diploma thesis 1992, University of Vienna; W. Pickl-Herck, diploma thesis 1995, University of Vienna; G. Wieser, diploma thesis 1995, University of Vienna, PhD Thesis, S. Schwaiger,. (2005) "Phytochemical, Pharmacological and Analytical Investigations of Edelweiss (Leontopodium alpinum Cass.) and related species". Leopold-Franzens-Universität Innsbruck, Austria).

In the literature (H. A. Hoppe, Drogenkunde (1975), Walter de Gryuter, Berlin, New York) *Leontopodium alpinum* is cited as "Herba leontopodii" with cardiac properties and as a remedy against dysentery and diarrhoea. J. L. Hartwell, Journal of Natural Products 31, (1968) 71-170, describes the use of *Leontopodium alpinum* against tumor diseases in Polish traditional medicine. Knowledge of the medicinal use of Edelweiss reaches back to past centuries: In historical literature *Leontopodium* was classified as a member of the genus *Gnaphalium* ("Ruhrkraut") and was used to treat angina, dysentery and breast cancer. *Leontopodium alpinium* has its origin in Asia, where about 31 different species can be found. Some of the Asian species have an almost identical appearance to the European species; some species show a growing shape like bushes or form needle-like leaves. Current theories suppose that during the last ice age the climate in the entire area between China and Europe was the same; as a consequence of this, Edelweiss spread from the Altai to the European mountain chains (for example Alps or Pyrenees). Apart from *L. alpinium* Cass. which grows in the Alpine region and most of the other European mountain chains, *L. nivale* Ten. (syn.: *L. alpinum* subsp. *nivale* Tutin) can be found very locally at the central Apennine.

In central Asia, *L. leontopodioides* BEAUVERD is widely distributed and is used in Tibetian medicine together with *L. dedejebsuu* BEAUVERD, *L. francheti* BEAUVERD, *L. palibinianum* BEAUVERD, *L. stracheyi* C. B. CLARKE *ex* HEMSL., and various *Anaphalis* species to treat diseases of the lymph nodes and poisoning with minerals and metals. In Mongolian folk medicine, *L. campestre* HAND.-MAZZ, *L. conglobatum* HAND.-MAZZ, *L. leontopodiodes,* and *L. ochroleucum* BEAUVERD are used for the therapy of cancer, diarrhoea, dysentery, heart disease, hepatitis and jaundice. Most diseases for which *Leontopodium* is traditionally used are infectious noxes.

However, the worldwide aging of the population has increased the incidents of cognitive deficits, such as the age-associated memory impairment and senile dementias and Alzheimer's disease, and the awareness of their disruptive impact on the life of the affected individuals. The "cholinergic hypothesis of learning" played a pivotal role in the development of drugs for Alzheimer's disease. At present, cholinesterase inhibition is the mainstay of treatment for Alzheimer's disease and serves as a promising strategy also for the treatment of senile dementia, ataxia, myasthenia gravis, vascular dementia, Lewy body dementia, Wemicke-Korsakoff-syndrome, and Parkinsons's disease.

A wide range of evidence shows that acetylcholinesterase (AChE) inhibitors can interfere with the progression of Alzheimer's disease. The successful development of these compounds was based on the theory that the decline in cognitive and mental functions associated with Alzheimer's disease is related to the loss of cortical cholinergic neurotransmission. These drugs increase the concentration of acetylcholine, a brain chemical (neurotransmitter) that helps nerve cells to communicate. The longer acetylcholine remains in the brain, the longer those cells can call up memories. The earliest known AChE inhibitors, namely, physostigmine and tacrine, showed modest improvement in the cognitive function of Alzheimer's patients. However, clinical studies show that physostigmine has poor oral activity, brain penetration and pharmacokinetic parameters while tacrine has hepatotoxic side effects. Studies were then focused on finding new types of acetylcholinesterase inhibitors that would overcome the disadvantages of these two compounds. Donepezil and Rivastigmin inaugurate a new class of AChE inhibitors with longer and more selective action with manageable adverse effects but still small improvement of cognitive impairment. Another inhibitor which has recently been approved for the treatment of Alzheimer's disease is galanthamine (Reminyl®), an alkaloid isolated from *Galanthus nivalis.* The alkaloid is selective, long acting, reversible and produces beneficial effects in patients. Huperzine A, a novel *Lycopodium* alkaloid discovered from the Chinese medicinal plant *Huperzia serrata* is a potent, reversible and selective inhibitor of AChE with a rapid absorption and penetration into the brain in animal tests. It exhibits memory-enhancing activities in animal and clinical trials. Compared to tacrine and donepezil, Huperzine A possesses a longer duration of action and higher therapeutic index, and the peripheral cholinergic side effects are less disturbing at therapeutic doses. At present, AChE inhibitors are the only effective therapy in Alzheimer's diseases with the exception of memantine which interferes with NMDA receptors and may inhibit excessive receptor stimulations due to enhanced extracellular glutamate concentration. AChE inhibitors have been found to be most helpful in earlier stages of the disease and can stop the progress of symptoms for up to 6 to 12 months. However, they do not cure or reverse the underlying dementia process.

Despite the substantial progress, the AChE inhibitors available are still not satisfactory. The scientific society is still in search for inhibitors with fast penetration in the CNS, long duration of action, reduced side effects and low toxicity. It is therefore the object of the present invention to provide AChE inhibitors which overcome the above mentioned disadvantages.

Surprisingly, it has been found, that extracts of the root of the genus *Leontopodium* and compounds isolated from the mentioned extracts exhibit *in vivo* a high activity in the treatment of degenerative diseases. Thus, the object of the present invention is solved by the provision of a plant extract from at least one *Leontopodium* species for the development of a medicament for the treatment of diseases which can be modulated by the selective inhibition of the enzyme acetylcholinesterase or by an increased acetylcholine concentration in the CNS. The plant extracts are particularly useful for the treatment of Alzheimer's disease.

The plant extracts can be obtained from the aerial parts of the plants as well as from the roots. Preferably, they are obtained from the roots of the *Leontopodium* plants.

Each known species of the genus *Leontopodium* can be used to prepare the extracts. The *Leontopodium* genus comprises:
*Leontopodium catipes* (DC.) F.Muell.
*Leontopodium gnaphalioides* Hieron.
*Leontopodium japonicum* var. *sandwicense* H.Lév.
*Leontopodium linearifolium* Britton
*Leontopodium meredithae* (F.Muell.) F. Muell.
*Leontopodium albogriseum* Hand.-Mazz.
*Leontopodium aloysiodorum* Hort. ex Hand.-Mazz.
*Leontopodium alpinum* Cass.
*Leontopodium alpinum* Colm. ex Willk. & Lange
*Leontopodium alpinum* Cass. subsp. *nivale* (Ten.) Tutin
*Leontopodium amrheinii* Hort. ex Mollers
*Leontopodium andersonii* C.B.Clarke
*Leontopodium antennarioides* Socz.
*Leontopodium arbuscula* Beauverd
*Leontopodium artemisiifolium* Beauverd
*Leontopodium aurantiacum* Hand.-Mazz.
*Leontopodium beerianum* Beauverd ex Murr
*Leontopodium blagoveshczenskyi* Vorosch.
*Leontopodium bonatii* Beauverd
*Leontopodium brachyactis* Gand.
*Leontopodium caespitosum* Beauverd
*Leontopodium caespitosum* Diels
*Leontopodium calocephalum* Beauverd
*Leontopodium campestre* Hand.-Mazz.
*Leontopodium catipes* F.Muell.
*Leontopodium chamaejasme* Beauverd
*Leontopodium charkeviczii* V. Yu. Barkalov
*Leontopodium chuii* Hand.-Mazz.
*Leontopodium conglobatum* Hand.-Mazz.
*Leontopodium coreanum* Nakai
*Leontopodium dedekensi* Beauverd
*Leontopodium delavayanum* Hand.-Mazz.
*Leontopodium discolor* Beauverd
*Leontopodium dubium* Beauverd
*Leontopodium evax* Beauverd
*Leontopodium fangingense* Ling
*Leontopodium fauriei* Hand.-Mazz.
*Leontopodium fedtschenkoanum* Beauverd
*Leontopodium fimbrilligerum* J.R.Drumm.
*Leontopodium fischerianum* Beauverd
*Leontopodium foliosum* Beauverd
*Leontopodium forrestianum* Hand.-Mazz.
*Leontopodium francheti* Beauverd
*Leontopodium futtereri* Diels
*Leontopodium giraldii* Diels
*Leontopodium gnaphalioides* Hieron. ex Sod.
*Leontopodium gracile* Hand.-Mazz.
*Leontopodium haastioides* Hand.-Mazz.
*Leontopodium hallaisanense* Hand.-Mazz.
*Leontopodium haplophylloides* Hand.-Mazz.
*Leontopodium hastatum* Beavera
*Leontopodium hayachinense* (Takeda) Hara & Kitam.
*Leontopodium helveticum* D.Don ex G. Don
*Leontopodium himalayanum* DC.
*Leontopodium* × *intermedium* Sunderm.
*Leontopodium jacotianum* Beauverd
*Leontopodium jacotianum* Beauverd var. *haastioides* (Hand.-Mazz.) R.C.Srivastava *Leontopodium jamesonii* Beauverd
*Leontopodium japonicum* Miq.
*Leontopodium japonicum* Miq. f. *happoense* Hid.Takah. ex T.Shimizu
*Leontopodium javanicum* Zoll. & Mor.
*Leontopodium junpeianum* Kitam.
*Leontopodium kamtschaticum* Komarov
*Leontopodium krasense* Derganc
*Leontopodium kurilense* Takeda
*Leontopodium leiolepis* Nakai
*Leontopodium leiolepis* Nakai var. *crinulosum* H.S.Pak
*Leontopodium leiolepis* Nakai var. *curvicollum* H.S.Pak
*Leontopodium leontopodinum* Hand.-Mazz.
*Leontopodium leontopodioides* Beauverd
*Leontopodium leontopodium* Karst.
*Leontopodium* × *lindavicum* Sunderm.
*Leontopodium linearifolium* Britton
*Leontopodium linearifolium* Benth. & Hook.f.
*Leontopodium linearifolium* Hand.-Mazz.
*Leontopodium longifolium* Ling
*Leontopodium* × *macranthum* Sunderm.
*Leontopodium maireanum* Beauverd ex Hand.-Mazz.
*Leontopodium makianum* Kitam.
*Leontopodium mariae* Muell.
*Leontopodium melanolepis* Ling
*Leontopodium meredithae* F.Muell.
*Leontopodium micranthum* Ling
*Leontopodium microcephalum* (Hand.-Mazz.) Ling
*Leontopodium microphyllum* Hayata
*Leontopodium monocephalum* Edgew.
*Leontopodium monoicum* Benth. & Hook.f.
*Leontopodium montisganeshii* S.Akiyama
*Leontopodium muscoides* Hand.-Mazz.
*Leontopodium nanum* Hand.-Mazz.
*Leontopodium nivale* (Ten.) Huet ex Hand.-Mazz.
*Leontopodium nivale* (Ten.) Huet ex Hand.-Mazz. subsp. *alpinum* (Cass.) Greuter *Leontopodium niveum* Hand.-Mazz.
*Leontopodium nobile* Beauverd
*Leontopodium ochroleucum* Beauverd
*Leontopodium ochroleucum* Beauverd subsp. *campestre* (Ledeb.) V.M.Khanminchun
*Leontopodium ochroleucum* Beauverd subsp. *campestre* (Hand.-Mazz.) Khanm.
*Leontopodium ochroleucum* Beauverd subsp. *conglobatum* (Turcz.) V.M.Khanminchun
*Leontopodium ochroleucum* Beauverd subsp. *conglobatum* (Hand.-Mazz.) Khanm.
*Leontopodium omeiense* Ling
*Leontopodium palibinianum* Beauverd
*Leontopodium paradoxum* J.R.Drumm.
*Leontopodium perniveum* Honda
*Leontopodium pirinicum* Hand.-Mazz.
*Leontopodium pulchellum* Beauverd
*Leontopodium pusillum* Hand.-Mazz.
*Leontopodium roseum* Hand.-Mazz.
*Leontopodium rosmarinoides* Hand.-Mazz.
*Leontopodium sachalinense* Miyabe & Kudo
*Leontopodium sandwicense* Rock
*Leontopodium shinanense* Kitam.
*Leontopodium sibiricum* Cass.
*Leontopodium sinense* Hemsl. ex Forb. & Hemsl.
*Leontopodium smithianum* Hand.-Mazz.
*Leontopodium souliei* Beauverd
*Leontopodium spathulatum* Kitam.
*Leontopodium stellatum* A.P.Khokhr.
*Leontopodium stoechas* Hand.-Mazz.
*Leontopodium stoloniferum* Hand.-Mazz.
*Leontopodium stracheyi* C.B.Clarke ex Hemsl.
*Leontopodium subulatum* Beauverd
*Leontopodium suffruticosum* Y.L.Chen
*Leontopodium tataricum* Kom.
*Leontopodium thomsonianum* Beauverd
*Leontopodium umbellatum* Bluff. & Fingerh.
*Leontopodium villosulum* A. P. Khokhr.
*Leontopodium villosum* Hand.-Mazz.
*Leontopodium wilsonii* Beauverd

Preferred species are *Leontopodium alpinum, Leontopodium sinense, Leontopodium campestre, Leontopodium leontopodioides, Leontopodium calocephalum, Leontopodium subulatum, Leontopodium dedekensii, Leontopodium artemiisifolium* and *Leontopodium francheti.*

Particular preferred are the species *Leontopodium alpinum, Leontopodium dedekensii, Leontopodium francheti* and *Leontopodium subulatum.*

As the starting material, dried aerial parts or dried roots of the *Leontopodium* species are used. The *Leontopodium* plant material is finely ground and then subjected to an extraction with different solvents. The solvents are selected with preference to their boiling points which should be in the range of 0°C to 150°C, preferably in the range of 20°C to 110°C. Suitable solvents are hexane, petroleum, benzene, chloroform, dichloromethane, diethylether, liquid carbon dioxide, ethanol and mixtures of water and alcohol. Preferably, dichloromethane and methanol are used as extraction solvent.

Alternatively to the extraction, the grounded plant material can be subjected to a water steam distillation to obtain the volatile constituents. The distillate can be used like the extract.

The extract may be obtained by extracting the roots with any of the solvents separately. It is further possible to subsequently extract the obtained extract with a second solvent.

The extraction can conveniently be conducted at room temperature. However, the extraction can also be conducted at an increased temperature up to the boiling point of the solvent used. Preferably, extraction of the plant material is performed at room temperature.

In order to obtain a high yield of the desired compounds, the solvent extraction is carried out two to ten times, preferably three to eight times.

The extracts are then combined and evaporated to dryness. In order to obtain the single compounds, the extract is further fractionated by column chromatography using silica gel, silica gel modified by means of AgNO₃, reversed phase material and Sephadex LH 20® as stationary phases. Additionally, other separation techniques e.g. high speed counter current chromatography are used as well.

The isolated compounds have been identified to belong to the groups of sesquiterpenes, coumarines, lignanes, dihydrobenzofuranes, polyacetylenes, diterpenes, and benzofuranes. The compounds are known per se.
Compounds of particular interest are:
a.) sesquiterpenes:
   Bisabolan A, bisabolan B, bisabolan C, bisabolan D, bisabolan E, isocomene, β-isocomene, silphinene, t-cadinol, 14-acetoxy-isocomene, 15-acetoxy-modphene, [(1 *R**,3a*S*,6*R*)-1,3a,6-trimethyl-1,3a,4,5,5a,6,7,8-octa-hydrocyclopenta[c]pentalen-2-yl]methylacetat, 6-acetoxy-3(15),7(14)-caryophylladiene, and isocomen-14-oate.
b.) coumarines:
   Obliquine, 5-hydroxy-obliquine, and 5-methoxy-obliquine.
c.) lignanes:
   [(2*S*,3*R*,4*R*)-4-(3,4-dimethoxybenzyl)-2-(3,4-dimethoxyphenyl)tetrahydrofuran-3-yl]methyl -(2*Z*)-2-methylbut-2-enoate, [(2*S*,3*R*,4*R*)-4-(3,4,t-trimethoxybenzyl)-2-(3,4,5-trimethoxyphenyl)tetrahydro-furan-3-yl]methyl-(2*Z*)-2-methylbut-2-enoate, and [(2*S*,3*R*,4*R*)-4-(3,4-dimethoxybenzyl)-2-(3,4,5-trimethoxyphenyl)tetrahydrofuran-3-yl)methyl-(2*Z*)-2-methylbut-2-enoate
d.) dihydrobenzofurane:
   1-{(2*R**,3*S**)-2-[1-(hydroxymethyl)vinyl]-3-[β-D-glucopyranosyloxy]-2,3-dihydro-1-benzo-furan-5-yl}-ethanone, 1-{(2*R**,3*R**)-3-(2-[(3*R*)-3-hydroxy-1-oxobutyl]-β-D-glucopyranosyloxy)-2,3-dihydro-2-[1-(methyl)vinyl]-1-benzofuran-5-yl}-ethanone, 1-{(2*R**,3*R**)-3-(β-D-glucopyranosyloxy)-2,3-dihydro-2-[1-(methyl)vinyl]-1-benzo-furan-5-yl}-ethanone, and 1-{(2*R**,3*R**)-2,3-dihydro-3-hydroxy-2-{1-[(β-D-glucopyranosyloxy)methyl] vinyl}-1-benzofuran-5-yl}-ethanone.
e). polyacetylenes:
   Polyacetylene(1-acetyloxy-3-angeloyloxy-(4*E*,6*E*)-tetradeca-4,6-diene-8,10,12-triyne and polyacetylene(1-acetyloxy-3-angeloyloxy-(4*E*,6*Z*)-tetradeca-4,6-diene-8,10,12-triyne.
f.) diterpenes:
   *ent*-kaur-16-enic-19-acid and (4a,7b,8a,9b,15a)-7,9-dihydroxy-15-{[(2*Z*)-2-methylbut-2-enoyl]oxy}kaur-16-en-19-oate.
g.) dihydrobenzopyrane:
   (S)-(-)-2,3-dihydro-2,6-dimethyl-4H-benzopyran-4-one.

The compounds are lipophilic and have a high penetration rate into the CNS system. Yet there is no rational statement possible conceming the potential of the compounds to exert peripheral side effects or toxicity. So far, these properties have not been tested in detail.

The *Leontopodium* extracts and/or any of the isolated compounds can be used for the treatment of diseases which can be modulated by the selective inhibition of the enzyme acetylcholinesterase or which show *in vivo* an increase of ACh in the CNS. In particular, the compounds and/or the extracts are suitable for treating Alzheimer's disease, vascular dementia, Lewy body dementia, Parkinsons's disease and Wernicke-Korsakoff-syndrome.

Alzheimers's disease is a neurodegenerative disorder that represents the most common cause of dementia in the elderly. The disease is caused by a loss of cholinergic neurons and their cholinergic projections from the basal forebrain area. The resulting reduction in cholinergic activity is correlated with the degree of cognitive impairment and is associated with decreased levels of the neurotransmitter acetylcholine and choline acetyltransferase (the rate-limiting enzyme for acetylcholine synthesis). The mainstay of treatment is inhibition of AChE. These drugs improve memory, cognitive and global function by augmenting cholinergic function and may also alter the natural course of the disease (Francis PT, Palmer AM, Snape M, Wilcock GK (1999) The cholinergic hypothesis of Alzheimer's disease: a review of progress. J Neurol Neurosurg Psychiatry, 66: 137-147; Lane RM, Kivipelto M, Greig NH (2004) The cholinergic hypothesis of Alzheimer's disease: a review of progress. Clin Neuropharmacol. 27: 141-149).

Vascular dementia (VaD) caused by stroke or small vessel disease, is the second most common form of dementia after Alzheimer's disease. (Goldsmith DR, Scott LJ, (2003) Donepezil: in vascular dementia. Drugs aging, 20: 1127-36;. Roman GC (2004) Facts, myths, and controversies in vascular dementia. Neurol Sci 226: 49-52). Experimental, clinical and pathological evidence has demonstrated low acetylcholine and reduced choline acetyltransferase (the acetylcholine-synthesizing enzyme) in the brain of VaD patients. Clinical trials show cognitive improvements by cholinergic drugs including acetylcholinesterase inhibitors.

Dementia with Lewy bodies (DLB) is a common cause of dementia. Changes in the acetylcholine system have been reported in brains of patients with DLB which provides a rational basis for trials of acetylcholinesterase inhibitors in DLB (Simard M. Van Reekum R. (2004) The acetylcholinesterase inhibitors for treatment of cognitive and behavioral symptoms in dementia with Lewy bodies. J Neuropsychiatry Clin. Neurosci. 16: 409-425).

Parkinson's disease (PD) is a progressive neurodegenerative disorder mainly characterized by degeneration of dopaminergic neurons in the substantia nigra and the ventral tegmental area. This process occurs in combination with a varying loss of central cholinergic (nucleus basalis of Meynert), noradrenergic (locus coeruleus) and serotonergic (dorsal raphe nuclei) integrity. Apart of the clinical motor hallmarks, 20-40% of patients develop also dementia, typically characterized by progressive dysexecutive syndrome with attentional deficit and fluctuating cognition, often accompanied by psychotic symptoms. These symptoms are thought to result mainly from dopaminergic deficiency in the nucleus caudatus (due to degeneration of projections from the substantia nigra and ventral tegmental area) and cholinergic deficiency in the cortex (due to degeneration of ascending projections from the nucleus basalis of Meynert).

Current treatment of dementia in PD is based on the compensation of profound cholinergic deficiency, as in recent studies with the cholinesterase inhibitors galantamine, donepezil and rivastigmine. It has also been shown that cholinesterase inhibitors can improve motor function in PD (Werber EA and Rabey JM (2001) The beneficial effect of cholinesterase inhibitors on patients suffering from Parkinson's disease and dementia. J Neural Transm, 108 p. 1319-1325).

Wemicke-Korsakoff syndrome is associated with a persisting severe anterograde amnesia in which memory is not transferred from short- to long-term storage. It is believed to be a consequence of a thiamine-deficient state often found in alcohol abusers. The memory deficit has been attributed to a number of brain lesions (corpus mamillare and dorsomedial nucleus of the thalamus), loss of cholinergic forebrain neurons and serotonin-containing neurons (Halliday G, Cullen K, Harding A (1994) Neuropathological correlates of memory dysfunction in the Wemicke-Korsakoff syndrome.Alcohol Alcohol Suppl. 2 p. 245-51). The studies and case-reports mentioned above suggest efficacy of acetylcholinesterase inhibitors in the Wemicke-Korsakoff-associated memory deficit.

The extracts and compounds of *Leontopodium* species were tested with regard to their inhibitory activity on the enzyme acetylcholinesterase. The following tests were used:

### In vitro studies

### Bioautographic test

The experiments are conducted by means of a bio-autographic test on TLC (thin layer chromatography)-plates applying a slightly modified method described by Rhee et al, Journal of Chromatography A 915 (2001) p. 217-223. Lyophilised acetylcholinesterase (commercial available) is dissolved in buffer A (Buffer A: 50 mM Tris-HCl, pH 8) to make a 1000 U/ml stock solution which is further diluted with buffer A to a concentration of 3 U/ml. The substrate acetylthiocholine iodide (ATCI) and the Ellman's reagent (DTNB, 5,5'-dithiobis-(2-nitrobenzoic acid)) are used in a concentration of 1 mM dissolved in buffer A, respectively. Stock solutions of the plant extracts (1 mg/ml MeOH) are prepared prior to application of 20 µl to the TLC-plates (TLC-alumina plates, 60F₂₅₄, 0.2 mm thickness). TLC is developed in the appropriate solvent system; enzyme inhibitory activities of the developed spots are detected by spraying the TLC plate first with the DTNB/ATCI reagent, whereas the silica should be saturated with solvent, and then allowed to dry for 3 to 5 minutes prior to the spraying of the enzyme solution. After applying the enzyme, a yellow background appears after about 5 minutes with white spots for possible AChE inhibiting compounds.

### Microplate Test

Experiments are carried out applying Ellman's method in a 96-well microplate assay described by Ingkaninan et al, Journal of Chromatography A 827 (2000) p. 6173 and Rhee et al, Journal of Chromatography A 915 (2001) p. 217-223. In addition to buffer A described above, a buffer containing 0.1 M NaCl and 0.02 M MgClx2.6 H₂O in buffer A is used for preparing a 3 mM solution of DTNB. For the microplate assay the substrate ATCI is dissolved 15 mM in water (HPLC grade). The enzyme stock solution is diluted with buffer A to get 0.22 U/ml. In the 96-well plates, 25 µl of 15 mM ATCl, 125 µl of 3 mM DTNB, 50 µl of buffer A, and 25 µl of sample (10 mM in MeOH, respectively DMSO (dimethylsulfoxide), diluted ten times with buffer A) are added. Final concentration of MeOH, respectively DMSO, comes to 1% in the well, which does not disturb the enzyme. Final concentration of tested samples is 100 µM. The absorbance of the product (2-nitrobenzoate-5-mercapto thioline) is measured at 405 nm with a Chameleon 5025 (Hidex, Finland) microplate reader 0, 15, 30, 45 and 60 minutes after adding 25 µl of 0.22 U/ml acetylcholinesterase-solution to the reaction mixture. All experiments are performed 4 times (n=4) and the data are statistically processed using the SPSS 11.5 program package. Percentage of inhibition is calculated by comparing the rates for the sample to the blank (10% MeOH, resp. DMSO, in buffer).

### In vivo studies

### Tests for increase in ACh release

*Surgery and push-pull technique:* Protocols of experiments were approved by the Bundesministerium für Wissenschaft, Forschung and Kunst, Austria, Kommission für Tierversuchsangelegenheitem. Sprague-Dawley rats (250-280 g) were anaesthetized with urethane 1.2 g/kg. i.p. The head was fixed in a stereotaxic frame and a push-pull cannula (outer tubing: o.d. 0.75 mm, i.d. 0.41 mm; inner tubing: o.d. 0.26 mm, i.d. 0.13 mm) was mounted on a microdrive. This was stereotaxically inserted through a hole in the skull into the nucleus accumbens (coordinates, mm from bregma: AP, +1.2, L, +2.5; V, -7.8) (Paxinos G. Watson H. (1998) The rat brain in stereotaxic coordinates, 4th edn. Academic Press, Sidney.). A second hole was drilled into the skull for the i.c.v. (intra-cerebroventricular) application of drugs (coordinates, mm from bregma: AP, -2.8; L, +1.5). The nucleus accumbens was superfused at 20µl/min with artificial cerebrospinal fluid (aCSF), pH 7.2. The CSF consisted of: NaCl,140; KCl, 3; CaCl₂, 1.2; MgCl₂, 1; Na₂HPO₄, 1; NaH₂PO₄, 0.3; glucose 3.0 (mmol 1⁻¹), neostigmine 1.0 (µmol/l⁻¹). After 1 h of the cannula insertion the samples were collected into tubes kept at -20° C in time periods of 10 min and then stored at -80°C until acetylcholine (ACh) was determined. *Application of drugs:* Drugs were dissolved in 20 µl of DMSO:EtOH 1:1. Using a motorized microsyringe connected to a stereotaxic microdrive, the tip was introduced with an angle of 60° through the drug application-hole into the ipsilateral ventricle (coordinates mm from bregma: AP, -0.9; L, +1.5; V, -3.8) via a cannula (o.d., 0.65; i.d., 0.38). The drug solutions were injected at 2µl/min. The control rats were injected with the vehicle (DMSO: EtOH 1:1).

*Histology:* At the end of the experiment the rats were killed with an overdose of urethane. A solution of cresyl violet was injected through the push-pull or i.c.v. cannulas. The brains were removed and stored in buffered formaldehyde solution (7%). Serial coronal sections were cut at 50 µm intervals. Localisations of the push-pull cannula and i.c.v. microinjection were verified on serial sections by the trace of the cannula and staining of the ventricle. Experiments with unsatisfactory localisation were discarded.

*Determination of acetylcholine (ACh):* ACh content in the samples was determined by HPLC with a cation-exchanging analytical column and a post-column reactor followed by electrochemical detection (Damsma et al, 1997; Prast et al; 1998). The system consisted of a HPLC-pump (JASCO PU-1580, Jasco Corporation, Tokyo, Japan), a pulse dampener and an autoinjector (AS-4000, Hitachi Ltd. Tokyo, Japan) with a 100-µL sample loop. The mobile phase was (mm): K₂HPO₄, 100; KCI, 5; tetramethylammonium hydroxide, 1, Na-EDTA, 0.1 and 0.5 mUL Kathon^{R} CG (Rohm & Haas, Frankfurt/Main, Germany), pH adjusted to 7.9 with H₂PO₄, pumped at a flow rate of 0.4 mL/min. ACh was separated on an analytical column (80 × 3 mm, Kromasil® 100-5 C18) loaded with laurylsulphate. At the post-column enzyme reactor (20 × 1 mm Kromasil® 100-10 NH₂) to which AChE and choline oxidase were bound, ACh was hydrolized to acetate and choline. Subsequently, choline was oxidized to betaine and hydrogen peroxide. The peroxide was electrochemically detected by a UniJet platin electrode (3mm) at +500 mV with an amperometric detector (BAS LC-4, Bioanalytical Systems, West Lafayette, IN, USA). The detection limit for ACh (signal to noise ratio = 3) was 10 fmol per sample. ACh was quantified with external standards that were injected at the beginning and the end of the analysis.

*Statistical analysis:* Statistical significance of drug effects on ACh release was calculated by Friedman's analysis of variance, followed by Wilcoxon's signed rank test for paired data using the mean release rate before the administration of drugs as controls. Data with n<6 were analysed with t-test for paired samples. Data are shown as means ± SEM of relative values. The mean release rate before drug administration was taken as 1.0.

### Behavioural studies: Tests for improvement of memory

*Animals:* C56BL/6N female, 3-month mice, weighing 20 - 27 g, were used in the present study. The animals were housed in groups of six to ten in cages (40 (L) x 25 (W) x 15 (H) cm) placed in a room at a constant temperature (22±1 °C), 12-light/ 12-dark cycle (07:00-19:00 h), with food and water *ad libitum.* Experiments were conducted between 09:00 and 16:00h.

*Surgery:* Mice were anaesthetized with Pentobarbital sodium (60 mg/kg, i.p.) and placed in a stereotaxic frame. A guide cannula (gauge 25, o.d. 0.52 mm, i.d, 0.26 mm and 7 mm long) was stereotaxically implanted through a hole on the skull into the brain area 0.5 mm higher than the third cerebral ventricle (-0.1 mm posterior, 0.8 mm lateral to bregma, -2.2 mm ventral to the dura) (Franklin KBJ, Paxinos G (1997) The mouse brain in stereotaxic coordinates. Academic Press San Diego). Guide cannula was secured to the skull using two small screws and dental cement. The microinjector (Hamilton gauge 32, o.d. 0.24 mm and i.d. 0.11 mm, 7.5 mm long) protruded 0.5 mm from the tip of the guide cannula to reach the cerebral ventricle. Mice recovered for 5 days before any experimentation was done.

*Drugs:* Scopolamine hydrobromide (Sigma, St. Louis, MO, U.S.A.) was dissolved in saline. Isocomene was dissolved in vehicle solution (3 parts DMSO : 7 parts sterile water). The animals were randomly divided into experimental groups and administered a cerebral ventricle injection of saline, scopolamine, isocomene or scopolamine and isocomene in a volume µl during 1 min. The injector was left in place an extra 1 min for drug diffusion after injection. These substances and vehicle were injected 15 min prior to the test.

*Experimental design and drug application:* Animals were randomly divided into three experimental groups (6-9 mice per group) as follows: vehicle; scopolamine hybrobromide 20 µg (50 nmol) in 1µl ICV, isocomene 8.16 µg (40 nmol) in 2µl ICV, and scopolamine hybrobromide 20 µg plus isocomene 2 µg ICV. All substances were injected 15 min before the test (T-maze and open field) or before the training trial T1 (object recognition test) through the 7.5 mm long injector connected with i.d. 0.28 mm polyethylene tubing to 5 µl Hamilton syringe.

### Object recognition task experiments:

*Apparatus:* The test apparatus consisted of a box made of Plexiglas (40 (L) x 25 (W) x 15 (H) cm), which was illuminated by a lamp above the box. In the different parts of the apparatus, the light intensity was equal at 100 lux. The objects to be discriminated were made of plastic or metal, were in the different shapes: cubes and cylinders 1.5-cm high in white or green colour; they could not be displaced by mice. In addition, these objects had no genuine significance for mice and had never been associated with a reinforcement.

Procedure: The object recognition test was performed as described elsewhere (Ennaceur A, Delacour J (1988) A new one-trial test for neurobiological studies of memory in rats. 1: Behavioural data. Behav Brain Res. 31: 47-59). On the day before testing, the animals were allowed to explore the apparatus for 10 min (without objects), while on the testing day, a session of two 10-min trials was given. During the training trial (sample trial) (T1), two identical objects were placed opposite sides 8 cm from the short walls. A mouse was placed in the middle of apparatus and was left to explore these two identical objects in 10 min. After T1, the mouse was put back in its home cage and a 24-hour inter-trial interval was given. Subsequently, the testing trial (choice trial) (T2), was performed in 10 min. During T2, a novel object (N) replaced one of the samples presented in T1, hence, the mouse was now exposed to two different objects: the familiar (F) and the novel one (N). All combinations and locations of objects were used in a balanced manner to reduce potential biases due to preferences for particular locations or objects. To avoid the presence of olfactory trails, the apparatus and the objects after each trial were thoroughly cleaned.

Exploration was defined as follows: directing the nose toward the object at a distance of less than 0.5 cm and/or touching the object with the nose. Turning around or sitting on the object was not considered as exploratory behavior. The times spent by mice in exploring each object during T1 and T2 were recorded manually by using a stopwatch. From this measure, a series of variables was then calculated: the total time spent in exploring the two identical objects in T1, and that spent in exploring the two different objects, familiar and novel in T2. The discrimination between the familiar and the novel object during T2 was measured by comparing the time spent in exploring the familiar sample with that spent in exploring the new object. As this time may be biased by differences in overall levels of exploration, a discrimination index (*D*) was then calculated; *D*=*N-F*/*N*+*F. D* is the discrimination ratio and represents the difference in exploration time expressed as a proportion of the total time spent exploring the two objects in T2.

### T-maze continuous alternation experiments

*Apparatus:* The T-maze was constructed according to the measures provided by Gerlai (Gerlai R. (1998) A new continuous alternation task in T-maze detects hippocampal dysfunction in mice. A strain comparison study. Behav. Brain Res 95: 91-101). The apparatus was made of grey plexiglas with guillotine doors at the start box and between the arms of the maze. The doors could be operated by the experimenter.

*Procedure:* Testing of a mouse consisted of one single session, which started with 1 forced-choice trial, followed by 14 free-choice trials.

In the first trial, the 'forced-choice trial', either the left or right goal arm is blocked by lowering the guillotine door. After the mouse has been released from the start arm, it will negotiate the maze, eventually enter the open goal arm, and retum to the start position. There, the animal will be confined for 5 s by lowering the guillotine door of the start arm.

During 14 'free-choice' trials, the mouse can choose freely between the left and right goal arm. After opening the guillotine door of the start arm the animal is free to choose between both goal arms (all guillotine doors open). As soon as the mouse has entered one goal arm, the other goal arm is closed. The mouse eventually retums to the start arm, and the next free-choice trial starts after a 5-s confinement in the start arm. A session is terminated and the animal is removed from the maze as soon as 14 free-choice trials have been performed or 30 min have elapsed, whatever event occurs first.

During the session, the animals are never handled by the experimenter. In order to isolate the cognitive component, care was taken to diminish the odour cues present by cleaning the maze with a damp sponge before each new animal started its session, as these cues are susceptible of influencing the alternation rate.

*Statistical analysis:* All data are shown as mean ± SEM. Statistical analysis was performed using the analysis of variance (ANOVA) followed by post hoc multiple comparisons with the Tukey-Kramer test.

### Examples

### Example 1

Dried roots of *L. alpinum* (specimen CH4 and CH5) were obtained from cultured sources. The ground roots of *L. alpinum* (804 g) were exhaustively macerated with 2.0 I dichloromethane (six times) and subsequently with 2.0 I methanol (six times) at room temperature. The extracts were evaporated to yield 14.7 g of the dichloromethane extract and 20.8 g of the methanol extract.

The extracts were investigated with respect to their AChE inhibitory activity using the microplate test described above. The results are shown in Table 1.

**Table 1: AChE inhibitory activity of Leontopodium alpinum extracts (concentration: 1 mg/ml).**

| **Sample** | **AChE inhibition (%) after 45 min (n = 3, mean** ± **S.D.)** |
|---|---|
| dichloromethane extract of Edelweiss roots (specimen CH5) | 78.79 ± 2.59 |
| methanol extract of Edelweiss roots (specimen CH5) | 51.81 ± 5.47 |
| dichloromethane extract of Edelweiss roots (specimen CH4) | 59.18 ± 2.83 |
| methanol extract of Edelweiss roots (specimen CH4) | 48.02 ± 8.03 |

### Example 2

Extracts of *L. dedekenesii, L. francheti and L subulatum* have been prepared according to Example 1 and tested with respect to their AChE inhibitory activity. The extracts were used in a concentration of 1 mg/ml. The results are shown in Table 2.

**Table 2: AChE inhibitory activity of different Leontopodium extracts (concentration: 1 mg/ml).**

| **Sample** | **AChE inhibition (%)** |
|---|---|
| dichloromethane extract of *L. dedekensii* | 35.3 ± 15.2 |
| methanol extract of *L. dedekensii* | 55.5 ± 15.2 |
| dichloromethane extract of *L. francheti* | 22.5 ± 7.5 |
| methanol extract of *L. francheti* | 37.4 ± 9.5 |
| dichloromethane extract of *L. subulatum* | 8.2 ± 7.3 |
| methanol extract of *L. subulatum* | 39.4 ± 10.5 |

### Example 3

Compounds listed in Table 3 were isolated as described by Dobner et al. 2003, Schwaiger et al. 2004 and Schwaiger 2004. (Dobner M.J., Ellmerer E.P., Schwaiger S., Narantuya S., Ododnchimeg B., Stütz M., Stuppner H. (2003): New lignan, benzofuran and sesquiterpene derivatives from the roots of Leontopodium alpinum and L. leontopodioides. Helvetica chimica acta 86(3), 733-738; Schwaiger, S., Adams, M., Seger, C., Ellmerer, E. P., Bauer, R., Stuppner, H. (2004): Novel constituents of Leontopodium alpinum Cass. and their in vitro leukotriene biosynthesis inhibitory activity. Planta Med. 70(10), 978-85; PhD Thesis, Schwaiger, S. (2005) "Phytochemical, Pharmacological and Analytical Investigations of Edelweiss (Leontopodium alpinum Cass.) and related species". Leopold-Franzens-Universität Innsbruck, Austria).

**Table 3: AChE inhibitory activity of constituents of Leontopodium species [100 µM] in the microplate assay after 60 min in comparison to medium control. Statistical analysis: ***p<0.001, **p<0.01, *p<0.05, Student's t-test of absorption data in comparison to medium control, n=4.**

| part A | | | part B | | |
|---|---|---|---|---|---|
| Compound | absorption x 10⁵ (mean ± sd) | inhibition (%) | compound | absorption x 10⁵ (mean ± sd) | inhibition (%) |
| Medium | 16477 ± 2759 | 0 | Medium | 13513 ± 3007 | 0 |
| 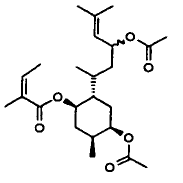 bisabolan A | 12599 ± 1728 | 23.3 | 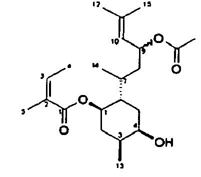 bisabolan E | 5811 ± 1238** | 56.9 |
| [(2*S*,3*R*,4*R* 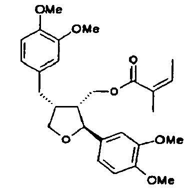 )-4-(3,4-dimethoxy-benzyl)-2-(3,4-dimethoxy-phenyl)tetrahydrofuran-3-yl]methyl-(2*Z*)-2-methylbut-2-en-oate | 13581 ± 2096 | 17.5 | 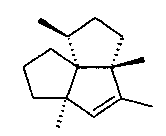 isocomene | 8063 ± 1344* | 39.6 |
| 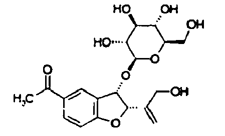 1-{(2*R**,3*R**)-2-[1-(hydroxymethyl)vinyl]-3-(β-D-gluco-pyranosyloxy)-2,3-dihydro-1-benzofuran-5-yl}-ethanone | 16496 ± 2091 | -0.6 | 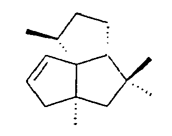 silphinene | 13024 ± 2561 | 3.2 |
| 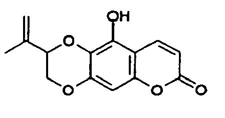 5-hydroxy-obliquin | 16085 ± 2083 | 1.9 | 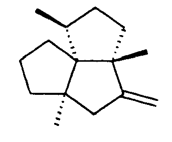 β -isocomene | 7958 ± 1539* | 40.8 |
| 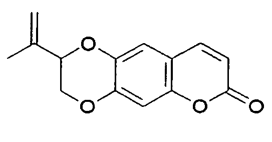 obliquine | 13450 ± 1956 | 18.1 | 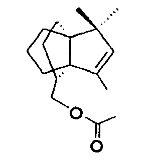 15-acetoxy-modhephene | 5891 ± 1237** | 56.4 |
| 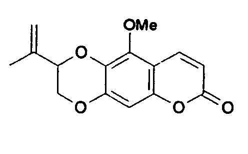 5-methoxy-obliquin | 15856 ± 2143 | 3.4 | 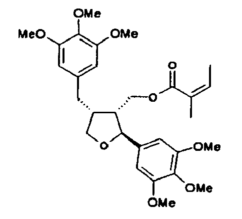 [(2S,3R,4R)-4-(3,4,5-trimethoxy-benzyl)-2-(3,4,5-trimethoxy-phenyl)tetrahydrofuran-3-yl]methyl-(2Z)-2-methylbut-2-en-oate | 14328 ± 2805 | -6.4 |
| 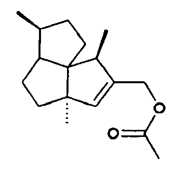 [(1R*,3aS,6R)-1,3a,6-trimethyl-1,3a,4,5,5a,6,7,8-octahydrocyclo-penta[c]pentalen-2-yl]methylacetat | 6288 ± 937** | 61.8 | 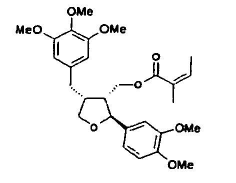 [(2S,3R,4R)-4-(3,4-dimethoxy-benzyl)-2-(3,4,5-trimethoxy-phenyl)tetrahydrofuran-3-yl]methyl-(2Z)-2-methylbut-2-en-oate | 12842 ± 2984 | 5.0 |
| 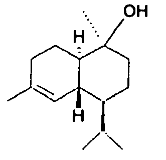 t-Cadinol | 14720 ± 1917 | 10.3 | 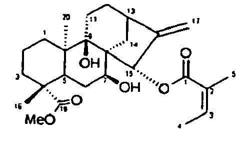 (4a,7b,8a,9b,15a)-7,9-dihydroxy-15-{[(2*Z*)-2-methylbut-2-enoyl]oxy} kaur-16-en-19-oate | 6241 ± 1352** | 53.7 |
| 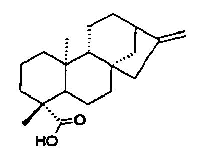 *ent*-kaur-16-enic-19-acid | 10559 ± 1391** | 35.6 | 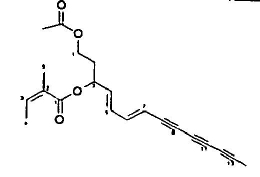 polyacetylene (1-acetyloxy-3-angeloyloxy-(4*E*,6*E*)-tetradeca-4,6-dien-8,10,12-triyne and the 4E,6Z isomer) | 11293 ± 2558 | 16.5 |

### Example 4

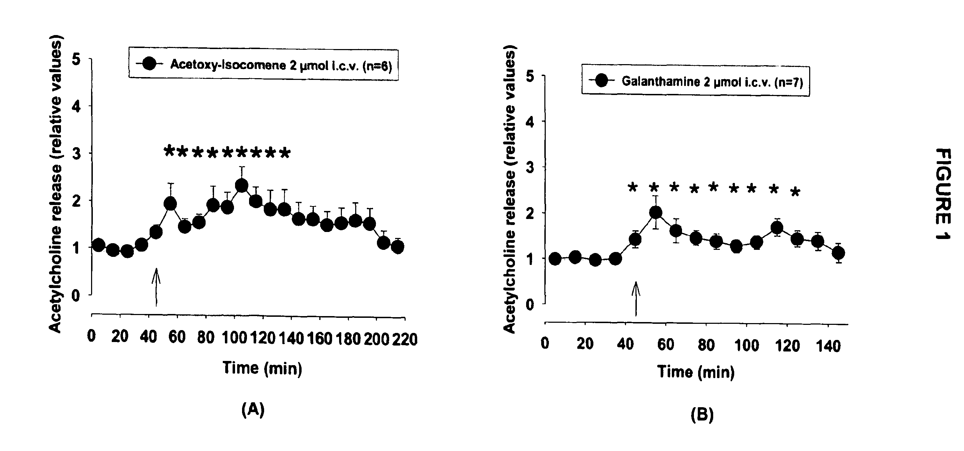

*In vivo* studies have been conducted as described above. The results indicate that the extracts and compounds according to the present invention exhibit an *in vivo* activity comparable to highly potent clinically tested compounds such as Galanthamin. Surprisingly, it has been found that even compounds showing no inhibitory activity in the *in vitro* studies, significantly enhance the concentration of neurotransmitters in the CNS. The respective results of 14-acetoxy-isocomene are shown below (Table 4 and Figure 1).

**Table 4: AChE inhibitory activity of 14-acetoxy-isocomene [100 mM] in the microplate assay after 60 min in comparison to medium control.**

| Compound | absorption x 10⁵ (mean ± sd) | inhibition (%) | |
|---|---|---|---|
| medium | 6521 ± 231 | 0 | |
| 14-acetoxy-isocomene | 6432 ± 521 | 1.4 ± 8.1 | c = 100 mM |

Figure 1 shows the effects of A) acetoxy-isocomene (2 µmol i.c.v.) and B) galanthamine (2 µmol i.c.v.) on release of ACh in rat brain. Galanthamine is shown for comparison. ACh release was determined in the rat nucleus accumbens with the push-pull technique. Statistical significance of drug effects was calculated by Friedman's analysis of variance followed by Wilcoxon's signed rank test for paired data (* p <0.05).

### Example 5

### Effects of isocomene on learning and memory in the object recognition and T-maze alternation tasks

Goal of the behavioural study was to show whether isocomene has the behaviour-influencing and memory-improving property capacities one would expect due to its in vivo AChE inhibitory effects.

The effect of isocomene on the behaviour of mice was tested in the object memory test and in the T-maze alternation test. It has been shown that inhibition of AChE exerts clear effects on the capabilities in learning and memory and behavioural performance of rodents in these tasks (Scali C, Giovannini MG, Prosperi C, Bartolini L, Pepeu G (1997) Tacrine administration enhances extracellular acetylcholine in vivo and restores the cognitive impairment in aged rats. Pharmacol Res 36: 463-9; Scali C, Giovanni MG, Prosperi C, Bartolini L, Hinz V, Schmidt P, Pepeu G (1997) Effect of metrifonate on extracellular brain acetylcholine and object recognition in aged rats. Eur J Pharmacol 325: 173-180; Spowart-Manning L, Van der Staay FJ, (2004) The T-maze continuous alternation task for assessing the effects of putative cognition enhancers in the mouse. Behav. Brain Res. 151:37-46). Experiments with injection of isocomene in mice pretreated with scopolamine are included within. This anticholinergic induces amnesia and behavioural alterations which are specifically antagonized by AChE inhibitors, thereby revealing their capacity to improve cholinergic deficits in the CNS.

### Effect of isocomene on scopolamine-induced learning-deficit in object recognition

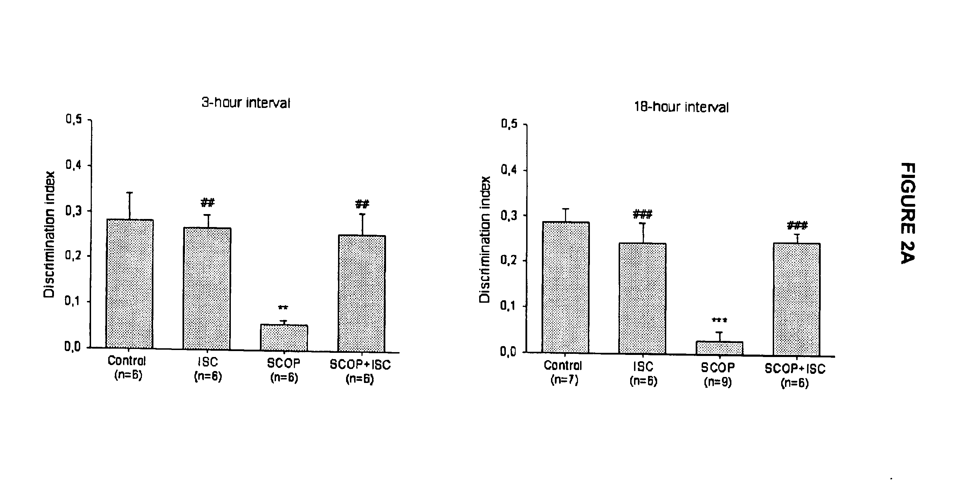

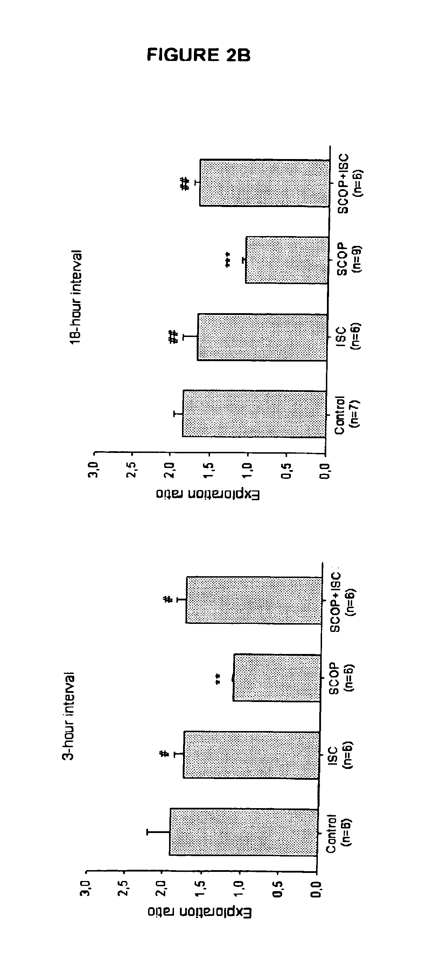

Scopolamine (20 µg ICV) induced a drastic impairment of learning. The mice of this group did not discriminate at all between the novel object and the familiar object in the T2, as indicated by the exploration ratio of close to 1.0 and the discrimination index of close to zero (Figure 4.1). The exploratory activity was not impaired since the exploration time showed no significant difference to that of vehicle-treated control mice (not shown). Isocomene (8.16 µg ICV) ameliorated this effect of scopolamine. It potently increased the exploration ratio and discrimination index indicating restoration of the scopolamine-impaired object recognition to a large extent (Figure 2A and Figure 2B).

Figure 2 shows the effect of isocomene (ISC; 8.16 µg ICV) on (A) the exploration ratio and the discrimination index (B) in the object recognition task. The cholinergic dysfunction is induced by scopolamine (SCOP; 20 µg ICV) (A). The discrimination index after a 3-hour and 18-hour interval is greatly reduced under influence of scopolamine but is restored by isocomene. (B) The exploration ratio is also reduced by scopolamine and ameliorated by isocomene. Data are shown as means ± SEM, analysis of variance ANOVA followed by multiple comparisons Tukey - Kramer test. ***P*<0.01, ****P*<0.001 vs control group; *^{#}P*<0.05, ^{##}*P*<0.01, ^{###}*P*<0.001 vs scopolamine group.

### Effect of isocomene on T-maze alternation

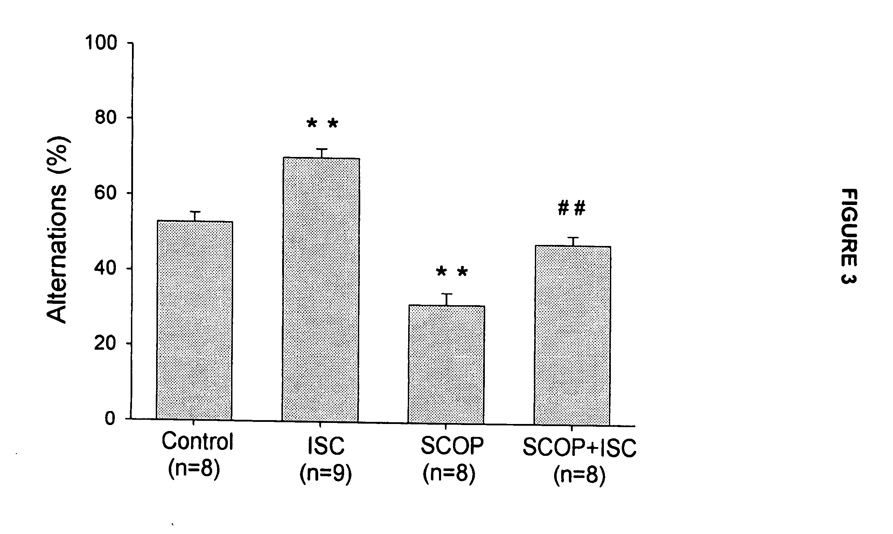

Each mouse was subjected to one forced choice trial followed by the free choice trials. Control mice selected the unfamiliar arm of the maze at a rate not significantly different from chance (52 ± 2.46 %, mean ± SEM). However, mice injected with 8.16 µg isocomene showed an increased alternation rate of 69.96 ± 2.42 % (mean ± SEM) which indicates a preference for entering the unfamiliar maze arm. These results suggest that isocomene facilitates the formation of the spatial working memory which is required to recognize the familiar part of the maze. In contrast to isocomene, the anticholinergic drug scopolamine induced a pronounced decrease in alternation rate. Isocomene abolished this effect of scopolamine (Figure 3).

Figure 3 shows the effect of isocomene on spontaneous alternation in the T-maze. Isocomene (8.16 µg ICV) increased the alternation rate and abolished the scopolamine-induced (20 µg ICV) memory impairment. Data are shown as means ± SEM., analysis of variance ANOVA followed by multiple comparisons Tukey - Kramer test. ***P*<0.01 vs control group; ^{##}*P*<0.01 vs scopolamine group.

The main findings of the behavioural study are that the isocomene ameliorates performance of mice in learning tasks in a way that is consistent with its presumed effect to inhibit brain AChE.

The object memory-paradigm reveals the ability of the animals to recognize the objects they have seen only once during the training trial. Normal mice have a natural propensity to explore novel objects, and when this bias is observed it is inferred that mice remember the familiar object. This kind of object recognition is based on episodic memory, a form of memory that is primarily affected in senile dementia and age-associated impairment and the Alzheimer type of dementia (Flicker C, Ferris SH, Crrok T, Bartus RT, Reisberg B (1985) Cognitive function in normal aging and early dementia. In: Traber J, Gispen, WH (eds) Senile dementia of the Alzheimer Type. Springer, Berlin Heidelberg New York pp 2-17; Lee AC, Rahman S, Hodges JR, Sahakian BJ, Graham KS (2003) Associative and recognition memory for novel objects in dementia: implications for diagnosis. Eur J Neurosci 18: 1660-70). Also in rats and mice, object discrimination appears to depend on the integrity of the cholinergic system. AChE inhibitors such as tacrine improve performance of the animals in the object memory task (Scali C, Giovannini MG, Prosperi C, Bartolini L, Pepeu G (1997) Tacrine administration enhances extracellular acetylcholine in vivo and restores the cognitive impairment in aged rats. Pharmacol Res 36: 463-9). The results indicate that object memory in mice is nearly abolished by ICV injection of 20 µg scopolamine. It has also been shown that even this strong inhibition by the anticholinergic is partially reversed by 8.16 µg isocomene, suggesting a potent action of isocomene on the brain cholinergic system.

Isocomene was also effective in the alternation task. When placed in a T-maze, rats or mice possess a strong tendency of alternating arm choices on successive trials. Spontaneous alternation rates have often been shown to be decreased by drugs which impair cholinergic transmission (Meyers B, Domino EF (1964) The effect of cholinergic drugs on spontaneous alternation in rats. Arch Int Pharmacodyn 150: 3-4; Kokkinidis L, Anisman H (1976) Interaction between cholinergic and catecholaminergic agents in a spontaneous alternation task. Psychopharmacology 48: 261-270). In contrast, AChE inhibitors increase alternation rate in untreated and pirenzepine-impaired animals (Delatour B, Gisquet-Verrier P (1996) Prelimbic cortex specific lesions disrupt delayed-variable response tasks in the rat. Behav Neurosci 110:1282-1298.; Egger GJ, Livesey PJ, Dawson RG (1973) Ontogenic aspects of central cholinergic involvement in spontaneous alternation behavior. Dev Psychobiol 6: 289-299; Ukai M, Shinkai N, Kameyama T (1995) Cholinergic receptor antagonists inhibit pirenzepine-induced dysfunction of spontaneous alternation performance in the mouse. Gen Pharmacol 26: 1529-1532). Consistent with these reported effects of AChE inhibitors, isocomene enhanced the alternation rate in untreated mice and reversed the scopolamine-induced decrease to control levels. Taken altogether, the most probable mechanism of action of isocomene on alternation behaviour is the inhibitory effect on brain AChE.

In conclusion, it has been shown that isocomene exerts potent influences on brain function. The results demonstrate the ability of the compound to ameliorate spontaneous and anticholinergic agent-induced learning deficits and confirm the therapeutic potential of the substance in the treatment of cognitive alterations caused by cholinergic hypofunction.

## Claims

1. Use of a plant extract from at least one Leontopodium species for the preparation of a medicament for the treatment of diseases which can be modulated by the selective inhibition of the enzyme acetylcholinesterase or by an increased acetylcholine concentration in the CNS when the disease is selected from Alzheimer's disease, vascular dementia , dementia with Lewis bodies, dementia associated with Parkinson's disease and the Wemicke-Korsakoff-Syndrome.

2. The use according to claim 1 wherein the disease is Alzheimer's disease.

3. The use according to any of claims 1 or 2 wherein *Leontopodium* species is selected from the group consisting of *Leontopodium alpinum, Leontopodium sinense, Leontopodium campestre, Leontopodium leontopodioides, Leontopodium calocephalum, Leontopodium subulatum, Leontopodium dedekensii, Leontopodium artemiisifolium* and *Leontopodium francheti.*

4. The use according to any of claims 1 to 3 wherein the extract is obtained from the roots of the Leontopodium species.

5. The use according to any of claims 1 to 4 wherein the extract is a dichloromethane extract.

6. The use according to any of claims 1 to 4 wherein the extract is a methanol extract.

7. The use of at least one compound selected from the group consisting of polyacetylene(1-acetyloxy-3-angeloyloxy-(4*E*,6*Z*)-tetradeca-4,6-diene-8,10,12-triyne,
*ent*-kaur-16-enic-19-acid,
(4a,7b,8a,9b,15a)-7,9-dihydroxy-15-{[(2*Z*)-2-methylbut-2-enoyl]oxy}kaur-16-en-19-oate,
(S)-(-)-2,3-dihydro-2,6-dimethyl-4*H*-benzopyran-4-one
for the preparation of a medicament for the treatment of diseases which can be modulated by the selective inhibition of the enzyme acetylcholinesterase or by an increased acetylcholine concentration in the CNS when the disease is selected from Alzheimer's disease, vascular dementia , dementia with Lewis bodies, dementia associated with Parkinson's disease and the Wemicke-Korsakoff-Syndrome.

## Patentansprüche

1. Verwendung eines Pflanzenextrakts aus mindestens einer Leontopodium-Art zur Herstellung eines Arzneimittels zur Behandlung von Erkrankungen, die durch die selektive Hemmung des Enzyms Acetylcholinesterase oder durch eine gesteigerte Acetylcholinkonzentration im ZNS beeinflusst werden können, wobei die Erkrankung aus Alzheimer-Krankheit, vaskulärer Demenz, Demenz mit Lewy-Körperchen, mit Parkinson-Krankheit verbundener Demenz und dem Wernicke-Korsakoff-Syndrom ausgewählt ist.

2. Verwendung gemäß Anspruch 1, wobei es sich bei der Erkrankung um die Alzheimer-Krankheit handelt.

3. Verwendung gemäß einem der Ansprüche 1 oder 2, wobei die *Leontopodium-Art* ausgewählt ist aus der Gruppe bestehend aus *Leontopodium alpinum, Leontopodium sinense, Leontopodium campestre, Leontopodium leontopodioides, Leontopodium calocephalum, Leontopodium subulatum, Leontopodium dedekensii, Leontopodium artemiisifolium* und *Leontopodium francheti*.

4. Verwendung gemäß einem der Ansprüche 1 bis 3, wobei der Extrakt aus den Wurzeln der Leontopodium-Art gewonnen wird.

5. Verwendung gemäß einem der Ansprüche 1 bis 4, wobei es sich bei dem Extrakt um einen Dichlormethan-Extrakt handelt.

6. Verwendung gemäß einem der Ansprüche 1 bis 4, wobei es sich bei dem Extrakt um einen Methanol-Extrakt handelt.

7. Verwendung von mindestens einer Verbindung, die ausgewählt ist aus der Gruppe bestehend aus:
Polyacetylen(1-acetyloxy-3-angeloyloxy-(4*E*,6*Z*)-tetradeca-4,6-dien-8,10,12-triin, *ent*-Kaur-16-en-19-säure,
(4a,7b,8a,9b,15a)-7,9-Dihydroxy-15-{[(2*Z*)-2-methylbut-2-enoyl]oxy}kaur-16-en-19-oat,
(S)-(-)-2,3-Dihydro-2,6-dimethyl-4*H*-benzopyran-4-on,
zur Herstellung eines Arzneimittels zur Behandlung von Erkrankungen, die durch die selektive Hemmung des Enzyms Acetylcholinesterase oder durch eine gesteigerte Acetylcholinkonzentration im ZNS beeinflusst werden können, wobei die Erkrankung aus Alzheimer-Krankheit, vaskulärer Demenz, Demenz mit Lewy-Körperchen, mit Parkinson-Krankheit verbundener Demenz und dem Wernicke-Korsakoff-Syndrom ausgewählt ist.

## Revendications

1. Utilisation d'un extrait végétal d'au moins une espèce de Leontopodium pour la préparation d'un médicament destiné au traitement de maladies qui peuvent être modulées par l'inhibition sélective de l'enzyme acétylcholinestérase ou par une concentration accrue d'acétylcholine dans le SNC quand la maladie est choisie parmi la maladie d'Alzheimer, la démence vasculaire, la démence à corps de Lewy, la démence associée à la maladie de Parkinson, et le syndrome de Wernicke-Korsakoff.

2. Utilisation selon la revendication 1, dans laquelle la maladie est la maladie d'Alzheimer.

3. Utilisation selon l'une quelconque des revendications 1 et 2, dans laquelle l'espèce de *Leontopodium* est choisie dans l'ensemble constitué par *Leontopodium alpinum, Leontopodium sinense, Leontopodium campestre, Leontopodium leontopodioides, Leontopodium calocephalum, Leontopodium subulatum, Leontopodium dedekensii, Leontopodium artemiisifolium* et *Leontopodium francheti.*

4. Utilisation selon l'une quelconque des revendications 1 à 3, dans laquelle l'extrait est obtenu à partir des racines de l'espèce de Leontopodium.

5. Utilisation selon l'une quelconque des revendications 1 à 4, dans laquelle l'extrait est un extrait au dichlorométhane.

6. Utilisation selon l'une quelconque des revendications 1 à 4, dans laquelle l'extrait est un extrait au méthanol.

7. Utilisation d'au moins un composé choisi dans l'ensemble constitué par
la polyacétylène(1-acétyloxy-3-angéloyloxy-(4*E*,6Z)-tétradéca-4,6-diène-8,10,12-triyne,
l'acide *ent*-kaur-16-énique-19,
le (4a,7b,8a,9b,15a)-7,9-dihydroxy-15-{[(2*Z*)-2-méthylbut-2-énoyl]oxy}kaur-16-én-19-oate,
la (S)-(-)-2,3-dihydro-2,6-diméthyl-4H-benzopyran-4-one, pour la préparation d'un médicament destiné au traitement de maladies qui peuvent être modulées par l'inhibition sélective de l'enzyme acétylcholinestérase ou par une concentration accrue d'acétylcholine dans le SNC quand la maladie est choisie parmi la maladie d'Alzheimer, la démence vasculaire, la démence à corps de Lewy, la démence associée à la maladie de Parkinson, et le syndrome de Wernicke-Korsakoff.
